(19) Europäisches Patentamt
European Patent Office
Office européen des brevets

(11) Veröffentlichungsnummer: **0 125 484**
**A1**

# (12) EUROPÄISCHE PATENTANMELDUNG

(21) Anmeldenummer: **84103951.4**

(22) Anmeldetag: **09.04.84**

(51) Int. Cl.³: **C 07 D 491/044**
C 07 D 495/04, A 61 K 31/40
A 61 K 31/55

(30) Priorität: **12.04.83 CH 1971/83**

(43) Veröffentlichungstag der Anmeldung:
**21.11.84 Patentblatt 84/47**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Blattner, Hans**
**Burgstrasse 116**
**CH-4125 Riehen(CH)**

(72) Erfinder: **Storni, Angelo, Dr.**
**Im Feuerbusch 3**
**CH-4310 Rheinfelden(CH)**

(74) Vertreter: **Zumstein, Fritz, Dr. et al,**
**Bräuhausstrasse 4**
**D-8000 München 2(DE)**

(54) **Polycyclische Carbonsäureverbindungen, Verfahren zu ihrer Herstellung und Präparate enthaltend solche Carbonsäureverbindungen, sowie deren Verwendung.**

(57) Verbindungen der Formel

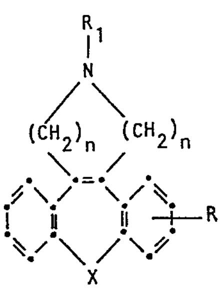

(I),

Worin R eine gegebenenfalls veresterte oder amidierte Carboxygruppe, $R_1$ Wasserstoff oder einen gegebenenfalls substituierten aliphatischen oder cycloaliphatisch-aliphatischen Kohlenwasserstoffrest, X Epoxy oder Epithio, und n 1 oder 2 bedeuten, oder Salze davon, weisen neuroleptische Verbindungen auf.

EP 0 125 484 A1

0125484



CIBA-GEIGY AG
Basel (Schweiz)

4-14383/+

Polycyclische Carbonsäureverbindungen, Verfahren zu ihrer Herstellung und Präparate enthaltend solche Carbonsäureverbindungen, sowie deren Verwendung

Die Erfindung betrifft polycyclische Carbonsäureverbindungen und Verfahren zu ihrer Herstellung, ferner pharmazeutische Präparate enthaltend solche Carbonsäureverbindungen und die Verwendung von letzteren als pharmakologisch wirksame Verbindungen.

Die Erfindung betrifft in erster Linie Verbindungen der Formel

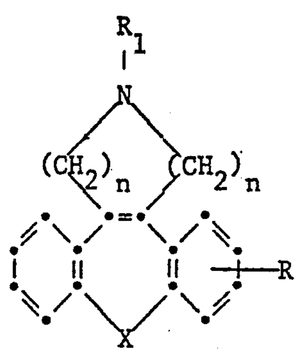

(I) ,

worin R eine gegebenenfalls veresterte oder amidierte Carboxygruppe, $R_1$ Wasserstoff oder einen gegebenenfalls substituierten aliphatischen oder cycloaliphatisch-aliphatischen Kohlenwasserstoffrest, X Epoxy oder Epithio, und n 1 oder 2 bedeuten, oder Salze von solchen Verbindungen.

Eine veresterte Carboxygruppe R ist in erster Linie Niederalkoxycarbonyl, während eine amidierte Carboxygruppe R insbesondere Carbamoyl oder Mono- oder Diniederalkyl-carbamoyl, ferner Niederalkylenaminocarbonyl, Oxaniederalkylenaminocarbonyl, Thianiederalkylenaminocarbonyl oder Azaniederalkylenaminocarbonyl darstellt, wobei Alkylenamino, Oxaalkylenamino, Thiaalkylenamino und Azaalkylenamino z.B. 4 bis 8 Ringatome aufweisen, und das Azastickstoffatom gegebenenfalls, z.B. durch gegebenenfalls substituiertes, wie gegebenenfalls veräthertes oder verestertes Hydroxy, z.B. Hydroxy, Niederalkoxy oder Niederalkanoyloxy, enthaltendes Niederalkyl substituiert sein kann.

Ein aliphatischer Kohlenwasserstoffrest ist in erster Linie Niederalkyl, kann jedoch auch Niederalkenyl oder Niederalkinyl sein, wobei sich in den ungesättigten Resten die Doppel- bzw. die Dreifachbindung in einer höheren als der 1-Stellung befindet. Ein cycloaliphatisch-aliphatischer Kohlenwasserstoffrest ist insbesondere Cycloalkylniederalkyl, worin Cycloalkyl z.B. bis und mit 8 Ringkohlenstoffatome enthalten kann. Substituenten von solchen Kohlenwasserstoffresten, die sich vorzugsweise in einer höheren als der 1-Stellung befinden, sind in erster Linie gegebenenfalls verätherte oder veresterte Hydroxygruppen, wie Hydroxy, Niederalkoxy, Niederalkanoyloxy oder Halogen.

Die Symbole n haben jeweils dieselbe Bedeutung und stehen entweder für 1 oder für 2.

Die vorstehend, sowie nachstehend verwendeten Allgemeindefinitionen haben, sofern nicht ausdrücklich anders angegeben, vorzugsweise folgende Bedeutungen:

Die mit "nieder" bezeichneten Gruppen und Verbindungen weisen in erster Linie bis und mit 7, vorzugsweise bis und mit 4 Kohlenstoffatome auf.

Niederalkoxycarbonyl ist z.B. Methoxycarbonyl oder Aethoxycarbonyl, ferner n-Propyloxycarbonyl, Isopropyloxycarbonyl, n-Butyloxycarbonyl,

Isobutyloxycarbonyl oder tert-Butyloxycarbonyl, sowie n-Pentyloxycarbonyl, n-Hexyloxycarbonyl oder n-Heptyloxycarbonyl.

Mono- und Diniederalkylcarbamoyl sind z.B. Methylcarbamoyl, Aethylcarbamoyl, Isopropylcarbamoyl, Dimethylcarbamoyl oder Diäthylcarbamoyl, während Alkylenaminocarbonyl, vorzugsweise mit 4 bis 8, z.B. mit 5 oder 6 Ringatomen, z.B. Pyrrolidinocarbonyl oder Piperidinocarbonyl, Oxaalkylenaminocarbonyl, vorzugsweise mit 6 Ringatomen, z.B. Morpholinocarbonyl, Thiaalkylenaminocarbonyl, vorzugsweise mit 6 Ringatomen, z.B. Thiomorpholinocarbonyl, und Azaalkylenaminocarbonyl, vorzugsweise mit 6 bis 8 Ringatomen, und gegebenenfalls mit substituiertem, z.B. Niederalkyl, Hydroxyniederalkyl oder Niederalkanoyloxyniederalkyl enthaltendem Azastickstoffatom, z.B. Piperazinocarbonyl oder 4-Methyl-piperazinocarbonyl bedeuten.

Niederalkyl ist z.B. insbesondere Methyl oder Aethyl, ferner n-Propyl, Isopropyl, n-Butyl, Isobutyl oder tert-Butyl, sowie n-Pentyl, Neopentyl, n-Hexyl oder N-Heptyl, während Niederalkenyl z.B. Allyl, 2-Methylallyl, 3,3-Dimethylallyl oder 2-Butenyl, und Niederalkinyl z.B. Propargyl bedeuten.

Cycloalkylniederalkyl ist z.B. Cyclopropylmethyl, Cyclobutylmethyl, Cyclopentylmethyl, 2-Cyclopentyläthyl, Cyclohexylmethyl, 1-Cyclohexyläthyl, 2-Cyclohexyläthyl, Cycloheptylmethyl oder Cyclooctylmethyl.

Niederalkoxy ist z.B. insbesondere Methoxy oder Aethoxy, ferner n-Propyloxy, Isopropyloxy, n-Butyloxy oder tert-Butyloxy.

Niederalkanoyloxy ist z.B. Acetyloxy, Propionyloxy oder Butyryloxy.

Halogen hat vorzugsweise eine Atomnummer bis zu 35 und ist z.B.

insbesondere Chlor, kann aber auch Fluor oder Brom sein.

Salze von Verbindungen der Formel I sind in erster Linie pharmazeutisch verwendbare Salze und können in erster Linie Säureadditionssalze sein, z.B. mit anorganischen Säuren, wie Halogenwasserstoffsäuren, z.B. Chlorwasserstoffsäure oder Bromwasserstoffsäure, ferner Salpetersäure, Schwefelsäure oder Phosphorsäure, oder mit organischen Säuren, wie entsprechenden Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, ferner Aminosäuren oder Sulfonsäuren, wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arensulfonsäure, z.B. Benzolsulfonsäure, p-Toluolsulfonsäure oder Naphthalin-2-sulfonsäure, oder mit anderen sauren organischen Substanzen, wie Ascorbinsäure. Verbindungen der Formel I, worin R für freies Carboxy steht, können auch Salze mit Basen, wie Salze mit Metallen, wie Alkali- oder Erdalkalimetall-, z.B. Natrium-, Kalium- oder Calciumsalze, oder mit organischen Basen, wie aliphatischen Aminen, z.B. Mono-, Di- oder Triniederalkylaminen, wie Diäthylamin oder Triäthylamin, oder Mono-, Di- oder Trihydroxyniederalkylaminen, wie Di-(2-hydroxyäthyl)-amin oder Tri-(2-hydroxyäthyl)-amin, oder innere Salze bilden.

Verbindungen der Formel I mit asymmetrischen Kohlenstoffatomen, z.B. in einem Rest $R_1$, können auch in Form von Isomeren, wie Racematen oder optisch aktiven Antipoden vorliegen.

Die neuen Verbindungen der Formel I und ihre Salze weisen pharmakologische, insbesondere neuroleptische Wirkungen auf. Diese basieren u.a. auf einer starken Hemmung der alpha-1-adrenergen Rezepto-

ren, die z.B. mittels eines Radiorezeptor-Tests anhand der Hemmung der ($^3$H) WB 4101-Bindung im Rattenhirn unter in vitro-Bedingungen, z.B. nach der von U'Prichard et al. in Molec. Pharmacol., Bd. 13, S. 454 (1977), beschriebenen Methode, und unter in vivo-Bedingungen, unter letzteren in Dosen ab etwa 0,01 mg/kg bei intraperitonealer Verabreichung an Ratten, nachgewiesen werden kann. Die neuen Verbindungen zeigen ebenfalls die für neuroleptisch wirksame Verbindungen charakteristische Hemmung der Dopaminrezeptoren, die z.B. anhand der Hemmung der ($^3$H) Spiperon-Bindung nach der von Bischoff et al. in Europ. J. Pharmacol., Bd. 68, S. 305 (1980), beschriebenen Methode in Dosen ab etwa 1 mg/kg bei intraperitonealer Verabreichung an Ratten festgestellt werden kann. Dabei zeigt es sich, dass die Hemmung der erfindungsgemässen Verbindungen ausgesprochen selektiv auf die hippokampalen Dopaminrezeptoren wirkt; sie ist z.B. mindestens dreimal stärker als diejenige auf die striatalen Dopaminrezeptoren.

Die neuen Verbindungen der Formel I und ihre Salze können deshalb als Neuroleptika, z.B. zur Behandlung von Schizophrenie, mit wesentlich geringeren extrapyramidalen Nebenwirkungen und einem günstigen Verhältnis zwischen erwünschter Wirkung und Toxizität und/oder unverwünschten Nebenwirkungen verwendet werden.

Die Erfindung betrifft in erster Linie Verbindungen der Formel I, worin R für Carboxy, Niederalkoxycarbonyl, Carbamoyl, Niederalkylcarbamoyl, Diniederalkylcarbamoyl, Niederalkylenaminocarbonyl mit 5 bis 8 Ringatomen, Oxaniederalkylenaminocarbonyl mit 6 Ringatomen,Thianiederalkylenaminocarbonyl mit 6 Ringatomen oder Azaniederalkylenaminocarbonyl mit 6 bis 8 Ringatomen steht, wobei der Azastickstoff gegebenenfalls durch Niederalkyl, Hydroxyniederalkyl, Niederalkoxyniederalkyl oder Niederalkanoyloxyniederalkyl substituiert sein kann, wobei in solchen substituierten Niederalkylresten ein Substituent durch mindestens 2 Kohlenstoffatome vom Azastickstoffatom getrennt ist, $R_1$ Wasserstoff, Niederalkyl, Hydroxyniederalkyl, Niederalkoxyniederalkyl, Niederalkanoyloxyniederalkyl, Halogenniederalkyl, Niederalkenyl, Niederalkinyl oder Cycloalkylniederalkyl bedeutet, wobei in substituierten Niederalkylresten ein Substituent vorzugsweise durch mindestens 2 Kohlenstoffatome vom Ringstickstoffatom getrennt ist, in ungesättigten Resten die Doppel- bzw. Dreifachbindung in höherer als der 1-Stellung vorliegt, und Cycloalkyl 3 bis 8 Ringatome enthält, wobei mit "nieder" bezeichnete Reste z.B. bis und mit 7, vorzugsweise bis und mit 4 Kohlenstoffatome enthalten, X Epoxy oder Epithio bedeutet, und n für 1 oder 2 steht, oder Salze, insbesondere pharmazeutisch verwendbare Salze, in erster Linie Säureadditionssalze von solchen Verbindungen.

Die Erfindung betrifft insbesondere Verbindungen der Formel I, worin R Carboxy oder Niederalkoxycarbonyl, in erster Linie jedoch Carbamoyl, ferner Niederalkylcarbamoyl oder Diniederalkylcarbamoyl, ferner Niederalkylenaminocarbonyl, worin Niederalkylenamino 5 oder 6 Ringatome enthält, bedeutet, wobei sich ein Rest R in Verbindungen der Formel I, worin n für 1 steht, vorzugsweise in 5-Stellung, und in Verbindungen der Formel I, worin n für 2 steht, vorzugsweise in 7-Stellung befindet, $R_1$ Wasserstoff, Niederalkyl, Hadroxyniederalkyl oder Niederalkoxyniederalkyl, worin Hydroxy bzw. Niederalkoxy durch mindestens 2 Kohlenstoffatome vom Ringstickstoffatom getrennt ist, Niederalkenyl oder Niederalkinyl, worin sich die Doppel- bzw. Dreifachbindung in einer höheren als der 1-Stellung befindet, oder Cycloalkylniederalkyl darstellt, worin Cycloalkyl 3 bis 8 Ringkohlenstoffatome enthält, wobei mit "nieder" bezeichnete Reste bis und mit 4 Kohlenstoffatome enthalten, X für Epoxy oder Epithio steht, und n 1 oder 2 bedeutet, oder Salze, insbesondere pharmazeutisch verwendbare Salze in erster Linie Säureadditionssalze von solchen Verbindungen.

Die Erfindung betrifft insbesondere Verbindungen der Formel

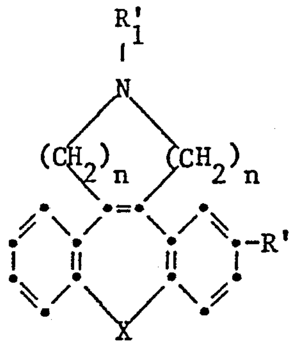

(Ia) ,

worin R' Carboxyl oder Niederalkoxycarbonyl, in erster Linie jedoch Carbamoyl, Niederalkylcarbamoyl oder Diniederalkylcarbamoyl, ferner Niederalkylenaminocarbonyl, worin Niederalkylenamino 5 oder 6 Ringatome enthält, bedeutet, $R_1'$ für Wasserstoff, Niederalkyl oder Cycloalkylniederalkyl steht, worin Cycloalkyl 3 bis 6 Ringkohlenstoffatome hat, wobei mit "nieder" bezeichnete Reste bis und mit 4 Kohlenstoffatome enthalten, X Epoxy oder Epithio bedeutet, und n für 1 oder 2 steht, oder Salze, insbesondere pharmazeutisch verwendbare Salze, in erster Linie Säureadditionssalze solchen Verbindungen.

Die Erfindung betrifft in erster Linie Verbindungen der Formel Ia, worin R' insbesondere für Carbamoyl, ferner für Niederalkylcarbamoyl, worin Niederalkyl bis und mit 4, vorzugsweise 1 oder 2 Kohlenstoffatome hat, z.B. Methylcarbamoyl, oder Diniederalkylcarbamoyl, worin Niederalkyl bis und mit 4, vorzugsweise 1 oder 2 Kohlenstoffatome hat, z.B. Dimethylcarbamoyl, ferner Alkylenaminocarbonyl, worin Alkylenamino 5 oder 6 Ringatome enthält, z.B. Pyrrolidinocarbonyl, steht, $R_1'$ Niederalkyl mit bis und mit 4, vorzugsweise mit 1 oder 2 Kohlenstoffatomen, z.B. Methyl, Aethyl oder n-Propyl, oder Cycloalkylniederalkyl, worin Cycloalkyl 3 bis 6, vorzugsweise 5 oder 6 Ringkohlenstoffatome aufweist, und Niederalkyl bis und mit 4, vorzugsweise 1 oder 2, Kohlenstoffatome hat, z.B. Cyclopropylmethyl, Cyclopentylmethyl oder Cyclohexylmethyl, X Epoxy oder vorzugsweise Epithio darstellt, und n vorzugsweise 1, ferner 2 bedeutet, oder Salze,

insbesondere pharmazeutisch verwendbare Salze, in erster Linie Säureadditionssalze von solchen Verbindungen.

Die Erfindung betrifft insbesondere die in den Beispielen beschriebenen Verbindungen.

Die neuen Verbindungen der Formel I können nach an sich bekannten Verfahren hergestellt werden, z.B. indem man

(a) in einer Verbindung der Formel

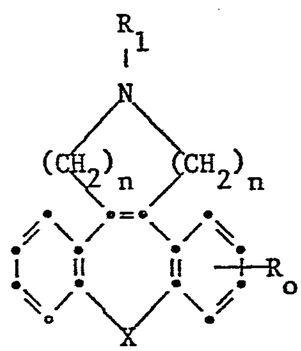

(II) ,

worin $R_o$ einen in die gegebenenfalls veresterte oder amidierte Carboxygruppe R überführbaren Rest darstellt, oder in einem Salz davon $R_o$ in die Gruppe R überführt, oder

(b) eine Verbindung der Formel

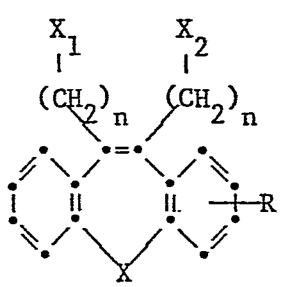

(III) ,

worin eine der Gruppen $X_1$ und $X_2$ den Rest der Formel $-NH-R_1$ (IIIa) und die andere eine reaktionsfähige veresterte Hydroxygruppe bedeutet, ringschliesst, und wenn erwünscht, eine erhältliche Verbindung der Formel I in eine andere Verbindung der Formel I überführt, und/oder, wenn erwünscht ein erhaltenes Salz in die freie Verbindung oder in ein anderes Salz überführt, und/oder, wenn erwünscht, eine erhaltene freie Verbindung in ein Salz überführt, und/oder, wenn erwünscht, ein erhaltenes Isomerengemisch in die einzelnen Isomeren auftrennt.

Salze von Ausgangsstoffen der Formel II sind in erster Linie Säureadditionssalze, wie entsprechende Salze mit starken Mineral- und organischen Sulfonsäuren, wie die entsprechenden Salze mit Chlorwasserstoff-, Schwefel-, Methansulfon- oder p-Toluolsulfonsäure.

Ein Rest $R_o$ ist insbesondere der Acylrest einer Carbonsäure, inklusive der entsprechende Rest eines Kohlensäurederivates. Ein Rest $R_o$ ist in erster Linie eine von der Gruppe R verschiedene, funktionell abgewandelte Carboxygruppe, wie z.B. eine in Anhydridform vorliegende Carboxygruppe, eine Thiocarbamoylgruppe, eine gegebenenfalls O-substituierte Formimidoylgruppe oder insbesondere die Cyangruppe. Die entsprechende Ausgangsstoffe der Formel II können in an sich bekannter Weise in die gewünschten Verbindungen der Formel I übergeführt werden.

Ein Ausgangsmaterial der Formel II mit einer anhydridisierten Carboxylgruppe kann das entsprechende symmetrische Anhydrid sein, ist jedoch vorzugsweise ein asymmetrisches Anhydrid, worin der Rest $R_o$ für eine, mit einer anorganischen oder organischen Säure anhydridisierte Carboxygruppe darstellt, wie Halogencarbonyl, insbesondere Chlorcarbonyl, oder Cyancarbonyl, ferner das, vorzugsweise in Salzform, z.B. in der Form des Lithiumsalzes, vorliegende Sulfo-oxycarbonyl, sowie Acyloxycarbonyl, worin Acyl z.B. den Rest einer aliphatischen oder aromatischen Carbonsäure bedeutet, und das besonders für Niederalkoxycarbonyloxycarbonyl, z.B. Aethoxycarbonyloxycarbonyl oder Isobutyloxycarbonyloxycarbonyl, steht.

Ausgangsstoffe der Formel II mit einer anhydridisierten Carboxygruppe $R_o$ können mittels Hydrolyse, z.B. durch Behandeln mit Wasser in saurem oder basischen Medium, in die entsprechenden freien Carbonsäuren oder mittels Alkoholyse, z.B. durch Behandeln mit einem Alkohol, wie Niederalkanol, in Gegenwart einer Base, wie eines Alkalimetallhydroxids, z.B. Natriumhydroxid, in die entsprechenden Ester übergeführt werden. Mittels Ammonolyse oder Aminolyse, z.B. durch Behandeln mit Ammoniak (gegebenenfalls in derivatisierter Form, z.B. als Harnstoff, Urethane, Formamid, oder Ammoniumsalze, wie Ammoniumacetat) bzw. mit Aminen (gegebenenfalls in derivatisierter Form, z.B. als N-substituierte Urethane) kann man aus Ausgangsstoffen der Formel II mit einer anhydridiesierten Carboxygruppe, insbesondere Chlorcarbonyl oder Niederalkoxycarbonyloxycarbonyl, als Rest $R_o$ entsprechende Verbindungen der Formel I mit einer amidierten Carboxygruppe R erhalten. Dabei kann man gegebenenfalls in Gegenwart eines säurebindenden Mittels, wie einer anorganischen oder organischen Base, z.B. eines Alkalimetall- oder Erdalkalimetallhydroxids, wie Natriumhydroxid, eines tertiären Amins, z.B. Triäthylamin, oder einer heteroaromatischen Base, z.B. Pyridin, oder eines Ueberschusses des Ammonolyse- bzw. Aminolysemittels arbeiten.

Eine anhydridisierte Carboxygruppe ist auch eine Trihalogenmethylgruppe, worin Halogen vorzugsweise Chlor ist, aber auch Brom sein kann, insbesondere Trichlormethyl. Eine Verbindung der Formel II mit einer solchen Gruppe $R_o$ stellt das gemischte Anhydrid einer, der Carbonsäureverbindung der Formel II entsprechenden Orthocarbonsäureverbindung mit einer Halogen-, insbesondere Chlorwasserstoffsäure dar. Ein entsprechendes Ausgangsmaterial kann mittels Hydrolyse, die vorzugsweise in Gegenwart einer Base, wie eines Alkalimetall- oder Erdalkalimetallhydroxids, z.B. Calciumhydroxid (gegebenenfalls in Gegenwart von Eisen), oder in Gegenwart eines geeigneten Schwermetallsalzes, wie Eisen-III-chlorid, in wässrigem Medium durchgeführt wird, in die entsprechende Carboxylverbindung der Formel I übergeführt werden. Man kann es auch durch Behandeln mit einem

mono- oder disubstituierten Amin, ferner auch mit Ammoniak, gegebenenfalls in derivatisierter Form, z.B. als Formamid oder Hexamethylentetramin, in eine Verbindung der Formel I umwandeln, worin R eine amidierte Carboxylgruppe darstellt.

Eine Thiocarbamoylgruppe $R_o$ in einem Ausgangsmaterial der Formel II kann eine unsubstituierte Thiocarbamoylgruppe sein, ist jedoch in erster Linie eine N-substituierte, wie N,N-disubstituierte Thiocarbamoylgruppe und insbesondere eine N,N-Niederalkylenaminocarbonylgruppe, worin die Niederalkylenkette z.B. durch ein Heteroatom, vorzugsweise durch Sauerstoff, unterbrochen sein kann; eine solche Thiocarbamoylgruppe $R_o$ ist vor allem Morpholinothiocarbonyl, ferner Thiomorpholinothiocarbonyl, sowie Piperidinothiocarbonyl. Ein Ausgangsmaterial der Formel I mit einer solchen Thiocarbamoylgruppe $R_o$ kann hydrolytisch, vorzugsweise in Gegenwart eines sauren Mittels, wie einer Mineralsäure, z.B. Schwefelsäure, oder eines basischen Reagens, wie eines Alkalimetallcarbonats oder -hydroxids, z.B. Natrium- oder Kaliumhydroxid, in die Verbindung der Formel I mit einer freien Carboxygruppe übergeführt werden. Durch Behandeln einer Thiocarbamoyl-Ausgangsstoffs der Formel II, worin Thiocarbamoyl $R_o$ unsubstituiert oder substituiert ist, mit einem Schwermetalloxid, z.B. Quecksilberoxid, oder einem basischen Mittel, wie einem Alkalimetall-, z.B. Natrium- oder Kaliumhydroxid, in einem Niederalkanol kann man zu entsprechenden Verbindungen der Formel I gelangen, worin R unsubstituiertes oder substituiertes Carbamoyl bedeutet.

In einem Ausgangsmaterial der Formel II ist eine gegebenenfalls O-substituierte Formimidoylgruppe $R_o$ insbesondere O-Niederalkyl-, wie O-Methyl- oder O-Aethyl-formimidoyl. Mittels Hydrolyse eines Säureadditionssalzes, wie des Hydrochlorids oder Sulfats, des Carboximidatausgangsmaterials der Formel II kann man die entsprechenden Ester, insbesondere Niederalkylester der Formel I erhalten, während man mittels Hydrolyse eines solchen Ausgangsmaterials der Formel II in Gegenwart einer Base, z.B. Natriumhydroxid, zu den Carbonsäureverbindungen der Formel I gelangen kann. Durch Erwärmen der oben erwähnten Säureadditionssalze von Carboximidatverbindungen der Formel II kann man zu den entsprechenden Verbindungen der Formel I mit einer amidierten Carboxygruppe gelangen, wobei Verbindungen der Formel II mit einer N-substituierten Formimidoylgruppe $R_o$ zu Verbindungen der Formel I führen, die eine entsprechend N-substituierte Carbamoylgruppe R aufweisen.

Die Ueberführung der bevorzugten Cyangruppe als Rest $R_o$ in einem Ausgangsmaterial der Formel II in eine gegebenenfalls veresterte oder amidierte Carboxygruppe erfolgt üblicherweise mittels Solvolyse, wobei man bei der Hydrolyse in Gegenwart einer Base, z.B. einer Alkalimetallhydroxids, wie Natrium- oder Kaliumhydroxid, oder einer Säure, z.B. einer Mineralsäure, wie Salzsäure, Schwefelsäure oder Phosphorsäure, zu Verbindungen der Formel I mit einer freien Carboxygruppe R gelangt.

Wird die Hydrolyse eines Cyanausgangsmaterials der Formel II unter besonderen, z.B. milderen, Bedingungen und insbesondere in Gegenwart von Wasserstoffperoxid durchgeführt, dann gelangt man durch Anlagerung von Wasser (Hydratisierung) zu Verbindungen der Formel I mit Carbamoyl als Gruppe R. Die Reaktion mit Wasserstoffperoxid, das üblicherweise im Ueberschuss vorliegt, kann in Gegenwart einer Base, wie eines Alkalimetall-, z.B. Natrium- oder Kaliumhydroxids, üblicherweise in einem wässrigen Medium, oder einer Säure, wie einer Mineral-, z.B. Chlorwasserstoff-, Schwefel- und/oder Phosphorsäure, üblicherweise in konzentrierter Form (letztere z.B. in Form von Polyphosphorsäure), oder einer Lewissäure, z.B. Bortrifluorid, gegebenenfalls in wässrigem oder wasserfreiem Medium (z.B. Chlorwasserstoffsäure in Eisessig), durchgeführt werden. Anstelle von Wasserstoffperoxid kann man auch den Komplex des Cyanausgangsmaterials der Formel II mit einem Bortrihalogenid, besonders Bortrichlorid, der hydrolytisch umgewandelt werden kann, verwenden, oder das Cyanausgangsmaterial der Formel II mit Ameisensäure, entweder allein bei hohen Temperaturen unter Druck oder in Gegenwart einer Mineralsäure, wie Chlor- oder Bromwasserstoffsäure, z.B. bei Raumtemperatur, behandeln und so zu Verbindungen der Formel I gelangen, worin R Carbamoyl bedeutet.

Ein Cyanausgangsmaterial der Formel II kann auch durch Behandeln mit einem Alkalimetall-, z.B. Kaliumhydroxid, vorzugsweise in fester Form, in einem Hydroxygruppen-haltigen Lösungsmittel, wie einem geeigneten Niederalkanol, z.B. tert-Butanol, und unter Erwärmen in eine Verbindung der Formel I mit Carbamoyl als Gruppe R, oder durch Behandeln mit einem Alkohol, z.B. einem Niederalkanol, insbesondere einem sekundären oder tertiären Niederalkanol, in Gegenwart einer Säure, wie einer Mineralsäure, z.B. wässriger Schwefelsäure, in eine Verbindung der Formel I mit einer entsprechend dem Alkohol, z.B. durch Niederalkyl, N-substituierten Carbamoylgruppe gelangen.

Durch Behandeln eines Ausgangsmaterials der Formel II, worin $R_o$ für Cyan steht, mit einem Alkohol, insbesondere einem Niederalkanol, in Gegenwart einer Säure, wie einer Mineralsäure, z.B. Schwefelsäure, und von Wasser kann man unter geeigneten Bedingungen zu Verbindungen der Formel I gelangen, worin R für eine veresterte Carboxygruppe steht; dabei wird intermediär einer der oben erwähnten Carboximidatausgangsstoffe der Formel II gebildet, worin $R_o$ für eine O-substituierte, insbesondere eine O-niederalkylierte Formimidoylgruppe steht.

Ferner kann man durch Behandeln einer Verbindung der Formel II, worin $R_o$ für den Acylrest einer organischen Carbonsäure, z.B. für Niederalkanoyl, wie Acetyl steht, mit Ammoniak in Gegenwart eines sauren Reagens, z.B. Schwefelsäure oder Polyphosphorsäure, zu Verbindungen der Formel I gelangen, worin R eine entsprechend N-substituierte, z.B. N-Alkyl-, wie N-Methyl-carbamoylgruppe darstellt.

In einem Ausgangsmaterial der Formel II kann der Rest $R_o$ auch für eine metallische Gruppe stehen und z.B. ein Alkalimetall-, wie Lithiumion, oder vorzugsweise ein Halogen-, wie Chlor- oder Brommagnesiumion darstellen. Ein entsprechendes Ausgangsmaterial kann durch Behandeln mit Kohlendioxid in eine Verbindung der Formel I umgewandelt werden, worin R für Carboxy steht.

Die oben erwähnten Reaktionen zur Umwandlung eines Restes $R_o$ in einem Ausgangsmaterial der Formel II in eine gegebenenfalls veresterte oder amidierte Carboxygruppe R kann in An- oder Abwesenheit von Lösungsmitteln, wobei ein geeignetes Reagens gleichzeitig auch als Lösungsmittel dienen kann, sowie von geeigneten Kondensationsmitteln und Katalysatoren durchgeführt werden, wobei man je nach Wahl des Ausgangsmaterials und der übrigen Reaktionsteilnehmer unter Kühlen oder Erwärmen, wenn notwendig, in einem geschlossenen System gegebenenfalls unter Druck und/oder in einer Inertgasatmosphäre arbeiten kann.

Die Ausgangsstoffe der Formel II sind bekannt oder können in an sich bekannter Weise hergestellt werden. So kann man sie z.B. nach dem unter (b) beschriebenen Verfahren erhalten, wenn man ein Ausgangsmaterial der Formel III einsetzt, worin R durch einen Rest $R_o$ oder einen anderen, in diesen überführbaren Rest darstellt, und letzteren dann in an sich bekannter Weise in diesen überführt. Ferner kann man in einem Ausgangsmaterial der Formel II, worin $R_o$ einen durch diesen Rest ersetzbaren Substituenten darstellt, einen solchen durch den Rest $R_o$ ersetzt. So kann z.B. eine Verbindung der Formel II, worin $R_o$ ein Halogenatom, wie Brom bedeutet, mit einem Alkalimetall-, z.B. Kalium-, oder einem Schwermetall-, z.B. Kupfer-I-cyanid, in einem geeigneten Lösungsmittel, wie einem Amid-artigen Lösungsmittel, z.B. Dimethylformamid, N-Methyl-pyrrolidin-2-on oder Hexamethylphosphorsäuretriamid, umgesetzt werden, und man erhält so Ausgangsstoffe der Formel II, worin $R_o$ für Cyan steht. Ferner

können in den Ausgangsstoffen der Formel II Reste $R_o$ in an sich bekannter Weise in andere Gruppen $R_o$ umgewandelt werden; so kann man z.B. Cyanverbindungen der Formel II ($R_o$ ist Cyan) durch Behandeln mit einem Alkohol in Gegenwart einer Säure, oder eine Niederalkanoyl-, z.B. Acetylverbindung der Formel II ($R_o$ ist Niederalkanoyl, z.B. Acetyl) z.B. durch Behandeln mit Ammoniumpolysulfid oder Schwefel in Gegenwart eines Amins, besonders eines N,N-disubstituierten Amins, wie N,N-Niederalkylenamin, worin die Niederalkylenkette gegebenenfalls durch ein Heteroatom, insbesondere ein Sauerstoffatom unterbrochen sein kann, und in erster Linie von Morpholin, bei erhöhter Temperatur, in eine entsprechende O-substituierte Carboximidat- bzw. Thiocarbamoylverbindung der Formel II ($R_o$ ist O-substituiertes Formimidoyl bzw. Thiocarbamoyl) umwandeln.

In einem Ausgangsmaterial der Formel III bedeutet eine reaktionsfähige veresterte Hydroxygruppe $X_1$ bzw. $X_2$ insbesondere eine mit einer starken Mineralsäure oder organischen Sulfonsäure veresterte Hydroxygruppe und steht z.B. für Halogen, wie Chlor oder Brom, oder für organisches Sulfonyloxy, wie Niederalkansulfonyloxy, z.B. Methansulfonyloxy, oder Arensulfonyloxy, z.B. p-Toluolsulfonyloxy. Der erfindungsgemässe Ringschluss kann in An- oder Abwesenheit eines Lösungsmittels oder Lösungsmittelgemisches, und/oder eines Kondensationsmittels, wie eines geeigneten säurebindenden Mittels, z.B. einer entsprechenden anorganischen Base, wie eines Alkalimetall-, z.B. Natriumhydroxids, oder organischen Base, wie eines tert-Amins, wie Triäthylamin, oder einer heteroaromatischen Base, z.B. Pyridin, ferner eines Ueberschusses einer Verbindung der Formel $H_2N-R_1$ (V) durchgeführt werden. Dabei kann man unter Kühlen oder Erwärmen, in einem geschlossenen Gefäss gegebenenfalls unter Druck und/oder unter einer Inertgasatmosphäre arbeiten.

Das Ausgangsmaterial der Formel III kann in an sich bekannter Weise hergestellt werden, wobei es unter den Reaktionsbedingungen direkt in die gewünschte Verbindung der Formel I übergeführt werden kann.

So kann man z.B. eine Verbindung der Formel

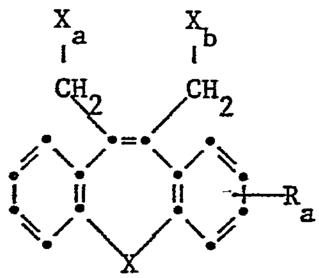

(IV) ,

worin $R_a$ für die Gruppe R, einen Rest $R_o$ oder eine in diesen überführbare Gruppe steht, und jeder der Reste $X_a$ und $X_b$ für Wasserstoff steht, durch Behandeln mit einer N-Halogen-amid, insbesondere N-Brom-succinimid, in eine Verbindung der Formel IV umwandeln, worin $X_a$ und $X_b$ für Halogen, besonders Brom stehen (Verbindung IVa). Diese kann, z.B. durch Behandeln mit einem geeigenten Cyansäuresalz, wie einem Ammoniumcyanid oder einem Alkalimetall-, z.B. Natrium- oder Kaliumcyanid, in eine Verbindung der Formel IV übergeführt werden, worin $X_a$ und $X_b$ Cyan bedeuten (Verbindung IVb). In dieser können die Cyangruppen, z.B. durch Behandeln mit einem Alkohol, wie einem Niederalkanol, z.B. Methanol oder Aethanol, in Gegenwart einer Säure, wie Chlorwasserstoffsäure, in veresterte Carboxy-, wie Niederalkoxycarbonylgruppen, z.B. Methoxycarbonyl oder Aethoxycarbonyl, und diese mittels Reduktion, d.h. durch Behandeln einer Verbindung der Formel IV, worin $X_a$ und $X_b$ für veresterte Carboxygruppen stehen (Verbindung IVc), z.B. mit einem Leichtmetallhydrid, wie Lithiumaluminiumhydrid, gegebenenfalls in Gegenwart von Aluminiumchlorid, in Carbinolgruppen umgewandelt werden (Verbindungen der Formel IV, worin $X_a$ und $X_b$ für Hydroxymethyl stehen; Verbindungen IVd). In einer Verbindung IVd können die Hydroxygruppen, z.B. durch Behandeln mit einem geeigneten Säurehalogenid, wie Thionylchlorid oder einem organischen Sulfonsäurechlorid,in reaktionsfähige veresterte Hydroxygruppen, z.B. Halogen bzw. organisches Sulfonyloxy, umgewandelt werden (Verbindungen der Formel IV, worin $X_a$ und $X_b$ für reaktionsfähige verestertes Hydroxymethyl stehen; Verbindungen IVe). Auf irgendeiner Stufe der Herstellung eines Zwischenproduktes der

Formel IVa oder IVe kann ein Rest $R_o$, gegebenenfalls nachdem er aus einer in ihn überführbaren Gruppe gebildet worden ist, in an sich bekannter Weise, z.B. nach einer der im Zusammenhang mit dem Verfahren (a) gezeigten Reaktionen, in die gewünschte Gruppe R umgewandelt werden. Eine Verbindung der Formel IVa oder IVe kann dann mit einem Amin der Formel $H_2N-R_1$ (V), gegebenenfalls in Gegenwart eines säurebindenden Mittels, wie einer der oben erwähnten Basen, behandelt werden, wobei man ein Ausgangsmaterial der Formel III erhalten kann, das, ohne isoliert zu werden, unter den Reaktionsbedingungen gegebenenfalls ringschliessen und direkt in die gewünschte Verbindung der Formel I übergeführt werden kann.

Erfindungsgemäss erhältliche Verbindungen der Formel I können in an sich bekannter Weise in andere Verbindungen der Formel I übergeführt werden. So kann man in Verbindungen der Formel I, worin R für eine veresterte oder amidierte Carboxygruppe steht, eine solche Gruppe R z.B. mittels Hydrolyse, z.B. in Gegenwart eines basischen Mittels, wie einer anorganischen Base, z.B. eines Alkalimetall- oder Erdalkalimetall-, z.B. Natrium-, Kalium- oder Calciumhydroxids, oder eines sauren Mittels, wie einer Mineralsäure, in eine freie Carboxygruppe R überführen.

Ferner kann man Verbindungen der Formel I, worin R für eine Carboxygruppe steht, z.B. durch Behandeln mit einem Alkohol, wie einem Niederalkanol, in Gegenwart eines geeigneten Veresterungsmittels, wie eines sauren Reagens, z.B. einer anorganischen oder organischen Säure, wie Chlorwasserstoff-, Trifluoressig- oder p-Toluolsulfonsäure, oder einer Lewissäure, z.B. Zinkchlorid, oder eines wasserbindenden Kondensationsmittels, z.B. eines Carbodiimids, wie N,N'-Dicyclohexyl-carbodiimid, oder durch Behandeln mit einem Diazoreagens, wie einem Diazoniederalkan, z.B. Diazomethan, in Verbindungen der

Formel I überführen, worin R für eine veresterte Carboxygruppe steht. Diese kann man auch erhalten, wenn man Verbindungen der Formel I, worin R für eine Carboxygruppe in freier Form oder in Salz-, wie Ammonium- oder Metall-, z.B. Alkalimetall-, wie Natrium- oder Kaliumsalzform vorliegt, mit einem reaktionsfähigen Ester eines Alkohols, wie Niederalkylhalogenid, z.B. Methyl- oder Aethylchlorid, -bromid oder -jodid, oder einem organischen Sulfonsäureester, wie einem organischen Sulfonsäureester, wie einem entsprechenden Niederalkylester, z.B. Methansulfonsäure- oder p-Toluolsulfonsäuremethylester oder -äthylester, behandelt.

Verbindungen der Formel I, worin R eine veresterte Carboxygruppe darstellt, kann man durch Umesterung, z.B. durch Behandeln mit einem Alkohol, üblicherweise einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetallniederalkanoats, -niederalkanolats oder -cyanids, wie Natriumacetat, -methanolat, -äthylat, -tert-butanolat oder -cyanid, oder eines geeigneten sauren Mittels, gegebenenfalls unter Entfernung des entstehenden Alkohols, z.B. durch Destillation, in andere Esterverbindungen der Formel I umwandeln. Man kann auch von sogenannten aktivierten Estern der Formel I ausgehen, worin R eine aktivierte veresterte Carboxygruppe darstellt (siehe unten), und diese durch Behandeln mit einem Alkohol, wie einem Niederalkanol, in einen anderen Ester umwandeln.

Die bevorzugten Amidverbindungen der Formel I, worin R für eine amidierte Carboxygruppe steht, können vorteilhaft auch aus den entsprechenden Säure- und Esterverbindungen der Formel I, worin R für eine gegebenenfalls veresterte Carboxygruppe steht, erhalten werden. So kann man z.B. Verbindungen der Formel I mit einer freien Carboxylgruppe R mit Harnstoff bei erhöhten Temperaturen, z.B. bei

200 - 240°, mit einem Formamid, z.B. Dimethylformamid, in Gegenwart eines geeigneten Kondensationsmittels, wie Phosphorpentoxid, bei erhöhten Temperaturen, oder mit einem Amin in Gegenwart eines geeigneten Kondensationsmittels, wie eines Carbodiimids, z.B. N,N'-Dihexyl-carbodiimid, ferner eines Phosphins, wie Triphenylphosphin (z.B. zusammen mit Bis-2-pyridyl-disulfid), oder eines Silans, wie Trichlorsilan (z.B. zusammen mit Pyridin) umsetzen, und die entsprechenden Amidverbindungen der Formel I, worin R für eine amidierte Carboxygruppe steht, erhalten. Sie können auch aus Verbindungen der Formel I, worin R für eine in Salzform vorliegende Carboxygruppe steht, erhalten werden, z.B. indem man ein entsprechendes Ammoniumsalz, z.B. durch Behandeln mit einem Dehydratisierungsmittel, wie Phosphorpentoxid, dehydratisiert, oder ein entsprechendes Alkalimetall-, z.B. Natriumsalz mit einem Amin, vorzugsweise in Gegenwart eines geeigneten Kondensationsmittels, wie Phenylphosphonsäuredichlorid, umsetzt.

Man kann Verbindungen der Formel I, worin R eine Carboxylgruppe darstellt, auch zuerst in ein reaktionsfähiges Derivat, wie ein Anhydrid, inkl. ein gemischtes Anhydrid, wie ein Säurehalogenid, z.B. -chlorid (z.B. durch Behandeln der Säureverbindung der Formel I, worin R für Carboxy steht, mit einem Thionylhalogenid, z.B. -chlorid), oder ein Anhydrid mit einem Ameisensäureester, z.B. -niederalkylester (z.B. durch Behandeln eines Salzes, wie eines Ammonium- oder Alkalimetallsalzes, der Säureverbindung der Formel I, worin R für Carboxy steht, mit einem Halogen-, wie Chlorameisensäureester, wie -niederalkylester), oder in einen aktivierten Ester, wie einen Cyanmethyl-, Nitrophenyl-, z.B. 4-Nitro-phenyl-, oder Polyhalogenphenyl-, z.B. Pentachlorphenylester (z.B. durch Behandeln einer Verbindung der Formel I, worin R für Carboxy steht, mit einer entsprechenden Hydroxyverbindung in Gegenwart eines geeigneten Kondensationsmittels, wie N,N'-Dicyclohexyl-carbodiimid) überführen, und ein solches reaktionsfähiges Derivat dann mit Ammoniak (gegebenenfalls in derivatisierter Form) oder einem Amin umsetzen und und so zu Amidverbindungen der Formel I gelangen, worin R für eine amidierte Carboxygruppe steht. Dabei kann man diese direkt oder über Zwischenverbindungen erhalten; so kann man z.B. einen aktivierten Ester, wie einen 4-Nitrophenylester, einer Verbindung der Formel I mit einer Carboxygruppe R zuerst mit einem 1-unsubstituierten Imidazol umsetzen und die so entstandene 1-Imidazolylcarbonylverbindung mit dem Ammoniak oder dem Amin in Reaktion bringen. Man kann auch andere, nicht-aktivierte Ester, wie Niederalkylester von Verbindungen der Formel I, worin R z.B. für Niederalkoxycarbonyl steht, mit Ammoniak oder Aminen zur Reaktion bringen und so Verbindungen der Formel I mit amidierten Carboxygruppen R bilden.

Die erfindungsgemäss bevorzugten Verbindungen der Formel I, worin R eine amidierte Carboxygruppe darstellt, können deshalb nach der Verfahrensvariante (a) dadurch hergestellt werden, dass man in einer Verbindung der Formel II, worin $R_o$ eine von einer amidierten Carboxygruppe R verschiedene Acylgruppe, insbesondere eine von einer amidierten Carboxygruppe R verschiedene, gegebenenfalls funktionell abgewandelte Carboxygruppe darstellt, den Rest $R_o$ in die Gruppe R überführt. Dabei kann die Acylgruppe Stickstoff-frei oder Stickstoff-haltig sein, und die bevorzugte, gegebenenfalls funktionell abgewandelte Carboxygruppe $R_o$ stellt entweder eine Stickstoff-freie, gegebenenfalls funktionell abgewandelte oder eine Stickstoff-haltige, funktionell abgewandelte Carboxygruppe dar. Generell werden die Verbindungen der Formel I mit einer amidierten Carboxygruppe R mittels Solvolyse dieser Ausgangsstoffe der Formel II erhalten, diejenigen mit einer Stickstoff-freien, gegebenenfalls funktionell abgewandelten Carboxygruppe vorzugsweise mittels einem der oben beschriebenen ammonolytischen oder aminolytischen und diejenigen mit einer Stickstoff-haltigen, funktionell abgewandelten Carboxygruppe mittels einem der hydrolytischen Verfahren.

In Verbindungen der Formel I, worin R für eine unsubstituierte oder N-monosubstituierte Carbamoylgruppe R steht, kann eine solche substituiert werden, z.B. durch Behandeln mit einem reaktionsfähigen Ester eines Alkohols, wie eines Niederalkanols, z.B. mit einem entsprechenden Halogenid oder einer entsprechenden organischen Sulfonyloxyverbindung, wie Niederalkylhalogenid, z.B. -chlorid, -bromid oder -jodid, oder Niederalkan- oder Aren-sulfonsäureniederalkylester, wobei vorzugsweise in Gegenwart eines basischen Mittels, wie eines Alkalimetall-, z.B. Natrium- oder Kaliumhydroxid gearbeitet wird. Dabei muss darauf geachtet werden, dass eine Quaternisierung des Ringstickstoffatoms vermieden wird.

Je nach Reaktionsbedingungen können die Verbindungen der Formel I in freier Form oder in Form von Salzen erhalten werden.

Erfindungsgemäss erhältliche Salze können in an sich bekannter Weise in die freien Verbindungen, Säureadditionssalze z.B. durch Behandeln mit einer Base, wie einem Alkalimetallhydroxid, Salze mit Basen z.B. durch Behandeln mit einer Säure, wie einer Mineralsäure umgewandelt werden. Salze, insbesondere Säureadditionssalze, können z.B. durch Behandeln mit geeigneten Derivaten, z.B. anorganischen Salzen von Säuren, in andere Salze umgewandelt werden.

Freie Verbindungen können, z.B. durch Behandeln mit Säuren oder entsprechenden Anionenaustauschern, oder freie Verbindungen der Formel I, worin R für Carboxyl steht, z.B. durch Behandeln mit geeigneten Basen in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Verbindungen, einschliesslich ihrer Salze, können auch in Form ihrer Hydrate erhalten werden, oder ihre Kristallformen können z.B. das zur Kristallisation verwendete Lösungsmittel einschliessen.

Die Verbindungen der Formel I können, je nach Substitution, auch in Form von Isomeren, z.B. von Racematen oder optischen Antipoden, erhalten werden.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Vorteilhafterweise isoliert man z.B. aus einem Racemat den pharmakologisch aktiveren Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, und/oder seiner Racemate bzw. Antipoden verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der vorliegenden Erfindung.

Die Erfindung betrifft ebenfalls die Verwendung der Verbindungen

der Formel I oder von pharmazeutisch verwendbaren Salzen von solchen Verbindungen, insbesondere als pharmakologisch, in erster Linie als neuroleptisch verwendbare Verbindungen. Dabei kann man sie, vorzugsweise in Form von pharmazeutischen Präparaten, in einem Verfahren zur prophylaktischen und/oder therapeutischen Behandlung des tierischen oder menschlischen Körpers, insbesondere zur Behandlung z.B. von Schizophrenie und anderen entsprechenden Erkrankungen verwenden.

Die Dosierung des Wirkstoffs, der allein oder zusammen mit dem üblichen Träger- und Hilfsmaterial verabreicht wird, hängt von der zu behandelnden Spezies, deren Alter und individuellen Zustand, sowie der Verabreichungsweise ab. Die täglichen Dosen liegen für Mammalien mit einem Körpergewicht von etwa 70 kg, je nach Art der Erkrankung, individuellem Zustand und Alter, vorzugsweise zwischen etwa 20 und etwa 300 mg.

Die Erfindung betrifft im weiteren pharmazeutische Präparate, die Verbindungen der Formel I oder pharmazeutisch verwendbare Salze von solchen Verbindungen als Wirkstoffe enthalten, sowie Verfahren zu ihrer Herstellung.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 10 bis etwa 400 mg, insbesondere von etwa 20 bis etwa 250 mg einer Verbindung der Formel I oder eines pharmazeutisch annehmbaren Salzes davon zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder

Calciumphosphate, z.B. Tricalciumphosphat oder Calciumhydrogenphosphat, ferner Bindemittel, wie Stärkekleister under Verwendung z.B. von Mais-, Weizen-, Reis- oder Kartoffelstärke, Gelatine, Traganth, Methylcellulose und/oder, wenn erwünscht, Sprengmittel, wie obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Stickkapseln können den Wirkstoff z.B. in Form eines Granulats, z.B. im Gemisch mit Füllstoffen, wie Lactose, Bindemitteln, wie Stärken, und/oder Gleitmitteln, wie Talk oder Magnesiumsetearat, und gegebenenfalls von Stabilisatoren enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstofffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässerige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren umfang in keiner Weise ein. Temperaturen werden in Celsiusgraden angegeben.

Beispiel 1: 38.7 g des Methansulfonsäuresalzes von 5-Cyan-2-methyl-2,3-dihydro-1H-dibenz[2,3:6,7]thiepino[4,5-c]pyrrol werden unter Rühren in 250 ml 85%ige Schwefelsäure bei Raumtemperatur eingetragen und anschliessend während 2 Stunden weitergerührt, wobei eine vollständige Lösung erzielt wird. Man lässt das Reaktionsgemisch 7 Tage bei Raumtemperatur stehen und tropft es dann vorsichtig unter Rühren in ein Gemisch von 800 ml einer 25%igen wässrigen Ammoniaklösung, Eis und etwa 1000 ml Essigsäureäthylester; durch Zugabe von Eis wird die Temperatur unter 30° gehalten. Hierauf wird die organische Phase abgetrennt, mit Wasser gewaschen, über Natriumsulfat getrocknet und auf ein kleines Volumen eingeengt, wobei das 2-Methyl-2,3-dihydro-1H-dibenz[2,3:6,7]thiepino[4,5-c]pyrrol-5-carboxamid, auskristallisiert; Smp. 170-174°.

Eine Lösung von 15.5 g des Produktes in 750 ml Aceton wird unter Rühren mit 4.8 g reiner Methansulfonsäure versetzt, worauf das Methansulfonsäuresalz von 2-Methyl-2,3-dihydro-1H-dibenz[2,3:6,7] thiepino[4,5-c]pyrrol-5-carboxamid auskristallisiert; Smp. 220-223°.

Das Ausgangsmaterial kann z.B. nach dem in der europäischen Patentanmeldung 79102146.2 (Publ. Nr. 7450) beschriebenen Verfahren hergestellt werden.

Analog dem obigen Verfahren können unter Verwendung der entsprechenden Ausgangsstoffe folgende Verbindung hergestellt werden:

2-(Cyclopentylmethyl)-2,3-dihydro-1H-dibenz[2,3:6,7]thiepino[4,5-c] pyrrol-5-carboxamid (amorphes Rohprodukt), dessen Methansulfonsäuresalz nach Umkristallisieren aus Methanol bei 248-251° schmilzt; aus 45.5 g des Methansulfonsäuresalzes von 5-Cyan-2-(cyclopentylmethyl)-2,3-dihydro-1H-dibenz[2,3:6,7]thiepino[4,5-c]pyrrol, das z.B. nach dem in der europäischen Patentanmeldung 79102146.2 (Publ. Nr. 7450) beschriebenen Verfahren hergestellt werden kann, in 250 ml 85 %iger Schwefelsäure und 800 ml einer 25 %igen wässrigen Ammoniaklösung;

2-Methyl-2,3-dihydro-1H-dibenz[2,3:6,7]oxepino[4,5-c]pyrrol-5-carboxamid, Smp. 218° nach Umkristallisieren aus Aceton; aus 32,9 g des Hydrochlorids von 5-Cyan-2-methyl-2,3-dihydro-1H-dibenz[2,3:6,7] oxepino[4,5-c]pyrrol in 250 ml 85 %iger Schwefelsäure und 800 ml einer 25 %igen wässrigen Ammoniaklösung; eine Lösung von 14.6 g des Produkts in einem Gemisch von 140 ml Aceton und 140 ml absolutem Aethanol wird mit 12.5 ml einer 4-n.Lösung von Chlorwasserstoff in absolutem Aethanol versetzt, worauf das Hydrochlorid von 2-Methyl-2,3-dihydro-1H-dibenz[2,3:6,7]oxepino[4,5-c]pyrrol-5-carboxamid auskristallisiert; Smp. 291-293°. Das Ausgangsmaterial kann z.B. durch Behandeln von 40.5 g 2-Cyan-10,11-bis-(brommethyl)-dibenz[b,f]oxepin mit 62 g Methylamin in 500 ml Methanol erhalten werden; das 5-Cyan-2-methyl-2,3-dihydro-1H-dibenz[2,3:6,7]oxepino[4,5-c]pyrrol schmilzt nach Kristallisieren aus Acetonitril bei 136-138°, und sein Methansulfonsäuresalz nach Kristallisieren aus absolutem Aethanol bei 220-223°;

2-Aethyl-2,3-dihydro-1H-dibenz[2,3:6,7]oxepino[4,5-c]pyrrol-5-carboxamid, Smp. 205-210° nach Umkristallisieren aus Essigsäureäthylester; aus 38.4 g des Methansulfonsäuresalzes von 2-Aethyl-5-cyan-2,3-dihydro-1H-dibenz[2,3:6,7]oxepino[4,5-c]pyrrol, das z.B. nach dem in der europäischen Patentanmeldung 79102146.2 (Publ. Nr. 7450) beschriebenen Verfahren hergestellt werden kann,in 250 ml 85 %iger Schwefelsäure und 800 ml einer 25 %igen wässrigen Ammoniaklösung; das Hydrochlorid des Produkts schmilzt nach Umkristallisieren aus absolutem Aethanol bei 215-220°; und

2-n-Propyl-2,3-dihydro-1H-dibenz[2,3:6,7]thiepino[4,5-c]pyrrol-5-carboxamid, Smp. 120 - 125° nach Kristallisieren aus Essigsäureäthylester; aus 41,4 g des Methansulfonsäuresalzes von 2-n-Propyl-5-cyan-2,3-dihydro-1H-dibenz[2,3:6,7]thiepino[4,5-c]pyrrol in 250 ml 85 %iger Schwefelsäure und 800 ml einer 25 %igen wässrigen Ammoniaklösung; das Methansulfonsäuresalz des Produkts schmilzt nach Kristallisieren aus absolutem Aethanol bis 252 - 255°. Das Ausgangsmaterial kann z.B. nach dem in der europäischen Patentanmeldung 79102146.2 (Publ. Nr. 7450) beschriebenen Verfahren hergestellt werden.

Beispiel 2: 41,5 g des Methansulfonsäuresalzes von 7-Cyan-3-methyl-2,3,4,5-tetrahydro-1H-dibenz[2,3:6,7]thiepino[4,5-d]azepin werden unter Rühren in 250 ml 85%iger Schwefelsäure bei Raumtemperatur eingetragen und anschliessend während 10 Stunden weitergerührt, wobei das Ausgangsmaterial vollständig in Lösung geht. Man lässt das Reaktionsgemisch 7 Tage bei Raumtemperatur stehen und tropft es dann vorsichtig unter Rühren in ein Gemisch von 800 ml einer 25%igen wässrigen Ammoniaklösung, Eis und etwa 1000 ml Essigsäureäthylester; durch Zugabe von Eis wird die Temperatur unter 30° gehalten. Hierauf wird die organische Phase abgetrennt, mit Wasser gewaschen, über Natriumsulfat getrocknet und auf ein kleines Volumen eingeengt, wobei das 3-Methyl-2,3,4,5-tetrahydro-1H-dibenz[2,3:6,7]thiepino[4,5-d]azepin-7-carboxamid auskristallisiert; Smp. 147 - 150°.

Eine Lösung von 16,8 g des Produkts in einem Gemisch aus 200 ml Aceton und 40 ml absolutem Aethanol wird mit 4,8 g reiner Methansulfonsäure versetzt, worauf das Methansulfonsäuresalz von 3-Methyl-2,3,4,5-tetrahydro-1H-dibenz[2,3:6,7]thiepino[4,5-d]azepin-7-carboxamid auskristallisiert; Smp. 220 - 223°.

Das Ausgangsmaterial kann z.B. nach dem in der deutschen Patentanmeldung P 27 23 105.6 beschriebenen Verfahren hergestellt werden.

Analog dem oben beschriebenen Verfahren können unter Verwendung der entsprechenden Ausgangsstoffe folgende Verbindungen hergestellt werden:

3-Methyl-2,3,4,5-tetrahydro-1H-dibenz[2,3:6,7]oxepino[4,5-d]azepin-7-carboxamid, Smp. 260-264° nach Kristallisieren aus Chloroform; aus 39.9 g des Methansulfonsäuresalzes von 7-Cyan-3-methyl-2,3,4,5-tetrahydro-1H-dibenz[2,3:6,7]oxepino[4,5-d]azepin in 250 ml 85 %iger Schwefelsäure und 800 ml einer 25 %igen wässrigen Ammoniaklösung; eine Lösung von 16 g des Produkts in 1500 ml absolutem Aethanol wird unter Rühren mit einer Lösung von 4.5 g wasserfreier Oxalsäure in 30 ml absolutem Aethanol versetzt, worauf das Oxalsäuresalz auskristallisiert; Smp. 240-242°. Das Ausgangsmaterial kann z.B. nach dem in der europäischen Patentanmeldung 80810377.4 (Publikation Nr. 30916) hergestellt werden;

3-(Cyclopentylmethyl)-2,3,4,5-tetrahydro-1H-dibenz[2,3:6,7]oxepino[4,5-d]azepin-7-carboxamid, Smp. 110-116° nach Kristallisieren aus Aceton; aus 46,7 g des Methansulfonsäuresalzes von 7-Cyan-3-(cyclopentylmethyl)-2,3,4,5-tetrahydro-1H-dibenz[2,3:6,7]oxepino[4,5-d]azepin in 250 ml 85 %iger Schwefelsäure und 800 ml einer 25 %igen wässrigen Ammoniaklösung; das Methansulfonsäuresalz des Produkts schmilzt nach Kristallisieren aus einem Gemisch von absolutem Aethanol und Aceton bei 176 - 180°. Das Ausgangsmaterial kann z.B. anch dem in der europäischen Patentanmeldung 80810377.4 (Publikation Nr. 30916) hergestellt werden; und

3-(Cyclopentylmethyl)-2,3,4,5-tetrahydro-1H-dibenz[2,3:6,7]thiepino

[4,5-d]azepin-7-carboxamid (amorphes Rohprodukt); aus 48,3 g des Methansulfonsäuresalzes von 7-Cyan-3-(cyclopentylmethyl)-2,3,4,5-tetrahydro-1H-dibenz[2,3:6,7]thiepino[4,5-d]azepin in 250 ml 85 %iger Schwefelsäure und 800 ml einer 25 %igen wässrigen Ammoniaklösung; eine Lösung von 20,2 g des Produkts in einem Gemisch von 200 ml Aceton und 50 ml absolutem Aethanol wird mit 12.5 ml einer 4-n.Lösung von Chlorwasserstoff in absolutem Aethanol versetzt, worauf das Hydrochlorid auskristallisiert, Smp. 228-232°. Das Ausgangsmaterial kann z.B. nach dem im Schweizer Patent 624105 beschriebenen Verfahren hergestellt werden.

Beispiel 3: 38,7 g des Methansulfonsäuresalzes von 5-Cyan-2-methyl-2,3-dihydro-1H-dibenz[2,3:6,7]thiepino[4,5-c]pyrrol werden unter Rühren in einem Gemisch von 310 ml Toluol und 160 ml Methanol gelöst. Die Lösung wird bei -10° bis -20° mit trockenem Chlorwasserstoff gesättigt und anschliessend während 20 Stunden bei 0 bis 5° weitergerührt. Hierauf lässt man 700 ml Eiswasser zufliessen und erwärmt während 1 Stunde bei 70-75°. Nach dem Abkühlen wird die wässerige Phase abgetrennt und mit einer konzentrierten wässrigen Ammoniaklösung alkalisch gestellt. Der als Oel ausgefallene 2-Methyl-2,3-dihydro-1H-dibenz[2,3:6,7]thiepino[4,5-c]pyrrol-5-carbonsäuremethylester wird mit Diäthyläther extrahiert, und der organische Extrakt mit Wasser gewaschen und nach dem Trocknen über Natriumsulfat unter vermindertem Druck eingedampft. Eine Lösung von 16.2 g des Rohprodukts in 100 ml Aceton wird mit 12,5 ml einer 4-n.Lösung von Chlorwasserstoff in absolutem Aethanol versetzt, worauf das Hydrochlorid auskristallisiert, das nach Umkristallisieren aus absolutem Aethanol bei 247-249° schmilzt.

Analog dem oben beschriebenen Verfahren können unter Verwendung der entsprechenden Ausgangsstoffe folgende Verbindungen hergestellt werden:

3-(Cyclopentylmethyl)-2,3,4,5-tetrahydro-1H-dibenz[2,3:6,7]oxepino[4,5-d]azepin-7-carbonsäuremethylester, Smp. 148-149° nach Kristallisieren aus Essigsäureäthylester; aus 46,7 g des Methansulfonsäuresalzes von 7-Cyan-3-(cyclopentylmethyl)-2,3,4,5-tetrahydro-1H-dibenz[2,3:6,7]oxepino[4,5-d]azepin in 310 ml Toluol und 160 ml Methanol und trockenem Chlorwasserstoff. Eine Suspension von 20.8 g des Produkts in 250 ml kochendem Aceton wird vorsichtig mit 4.8 g reiner Methansulfonsäure versetzt, wobei eine vollständige Lösung entsteht, aus der nach kurzer Zeit das Methansulfonsäuresalz von 3-(Cyclopentylmethyl)-2,3,4,5-tetrahydro-1H-dibenz[2,3:6,7]oxepino[4,5-d]azepin-7-carbonsäuremethylester auskristallisiert, Smp. 229-231°;

3-Methyl-2,3,4,5-tetrahydro-1H-dibenz[2,3:6,7]oxepino[4,5-d]azepin-7-carbonsäuremethylester, Smp. 130-131° nach Kristallisieren aus Aceton; aus 39,9 g des Methansulfonsäuresalzes von 7-Cyan-3-methyl-2,3,4,5-tetrahydro-1H-dibenz[2,3:6,7]oxepino[4,5-d]azepin in 310 ml Toluol und 160 ml Methanol und trockenem Chlorwasserstoff; das Methansulfonsäuresalz schmilzt nach Kristallisieren aus absolutem Aethanol bei 230-233°; und

3-Methyl-2,3,4,5-tetrahydro-1H-dibenz[2,3:6,7]thiepino[4,5-d]azepin-7-carbonsäuremethylester, Smp. 133-134° nach Kristallisieren aus Diäthyläther; aus 41,5 g des Methansulfonsäuresalzes von 7-Cyan-3-methyl-2,3,4,5-tetrahydro-1H-dibenz[2,3:6,7]thiepino[4,5-d]azepin in 310 ml Toluol und 160 ml Methanol und trockenem Chlorwasserstoff; das Methansulfonsäuresalz schmilzt nach Kristallisieren aus absolutem Aethanol bei 224-225°.

Beispiel 4: 40,4 g 3-(Cyclopentylmethyl)-2,3,4,5-tetrahydro-1H-dibenz[2,3:6,7]oxepino[4,5-d]azepin-7-carbonsäuremethylester werden mit einer Lösung von 7 g Natriumhydroxyd in 150 ml 70%igem wässrigem Aethanol unter Rühren 1 Stunde am Rückfluss gekocht. Anschliessend wird das heisse Reaktionsgemisch mit 450 ml destilliertem Wasser verdünnt und mit 300 ml 10%iger Methansulfonsäure versetzt; der entstandene Niederschlag geht durch Erwärmen auf 80-90° wieder in Lösung. Durch Zugabe von konzentrierter wässriger Ammoniaklösung bis pH 8-9 fällt die 3-(Cyclopentylmethyl)-2,3,4,5-tetrahydro-1H-dibenz[2,3:6,7]oxepino[4,5-d]azepin-7-carbonsäure aus, welche abgenutscht und mit Wasser und Aethanol gewaschen wird, Smp. 260-262° nach dem Trocknen unter vermindertem Druck bei 100°; das Methansulfonsäuresalz schmilzt bei etwa 215° (unter Zersetzung).

In analoger Weise können bei Auswahl der geeigneten Ausgangsstoffe folgende Verbindungen erhalten werden:

2-Methyl-2,3-dihydro-1H-dibenzo[2,3:6,7]thiepino[4,5-c]pyrrol-5-carbonsäure, Smp. 194-198°; aus 32,3 g 2-Methyl-2,3-dihydro-1H-dibenzo[2,3:6,7]thiepino[4,5-c]pyrrol-5-carbonsäuremethylester und 7 g Natriumhydroxyd in 150 ml 70%igem wässrigem Aethanol;

3-Methyl-2,3,4,5-tetrahydro-1H-dibenz[2,3:6,7]oxepino[4,5-d]azepin-7-carbonsäure, Smp. 280-288°; aus 33.5 g 3-Methyl-2,3,4,5-tetrahydro-1H-dibenz[2,3:6,7]oxepino[4,5-d]azepin-7-carbonsäureester und 7 g Natriumhydroxyd in 150 ml 70%igem wässrigem Aethanol; das Methansulfonsäuresalz des Produkts schmilzt nach Kristallisieren aus Aethanol bei 310-314°; und

3-Methyl-2,3,4,5-tetrahydro-1H-dibenz [2,3:6,7]thiepino[4,5-d]azepin-7-carbonsäure, Smp. etwa 295-305° (unter Zersetzung); aus 35,1 g

3-Methyl-2,3,4,5-tetrahydro-1H—dibenz [2,3:6,7]thiepino[4,5-d]azepin-7-carbonsäuremethylester und 7 g Natriumhydroxyd in 150 ml 70%igem wässrigem Aethanol; das Methansulfonsäuresalz des Produkts schmilzt nach Kristallisieren aus einem Gemisch von Aethanol und Diäthyläther bei 200-203°.

Beispiel 5: Ein Gemisch von 38,9 g 3-(Cyclopentylmethyl)-2,3,4,5-tetrahydro-1H-dibenz[2,3:6,7]oxepino[4,5-d]azepin-7-carbonsäure, 1200 ml Methylenchlorid und 15,2 g Triäthylamin wird unter Rühren 30 Minuten am Rückfluss gekocht, anschliessend auf -10° abgekühlt und innerhalb von 30 Minuten mit einer Lösung von 16,3 g Chlorameisensäureäthylester in 50 ml Methylenchlorid tropfenweise versetzt. Nach Beendigung der Zugabe lässt man die klare Reaktionslösung eine Stunde bei -10 bis 0° nachrühren und tropft hierauf bei dieser Temperatur eine Lösung von 6,8 g Dimethylamin in 50 ml Methylenchlorid zu; durch zweistündiges Rühren bei Raumtemperatur wird die Reaktion zu Ende geführt. Das Reaktionsgemisch wird mit Wasser und 2-n. wässriger Natriumhydroxidlösung gewaschen und mit einer 5%igen Lösung von Methansulfonsäure in Wasser extrahiert. Der saure Extrakt wird mit konzentrierter wässriger Natriumhydroxidlösung alkalisch gestellt, wobei das 3-(Cyclopentylmethyl)-2,3,4,5-tetrahydro-1H-dibenz[2,3:6,7]oxepino[4,5-d]azepin-7-carbonsäure-N,N-dimethylamid auskristallisiert, das nach dem Trocknen unter rereduziertem Druck bei 80° und anschliessendem Umkristallisieren aus Aceton bei 142-144° schmilzt.

Eine Lösung von 20,8 g des Produkts in 200 ml Aceton wird unter Rühren mit 4,8 g reiner Methansulfonsäure versetzt, worauf das Methansulfonsäuresalz auskristallisiert; Smp. 264-268°.

Analog dem oben beschriebenen Verfahren können unter Verwendung der entsprechenden Ausgangsstoffe folgende Verbindungen hergestellt werden:

3-(Cyclopentylmethyl)-2,3,4,5-tetrahydro-1H-dibenz[2,3:6,7]oxepino [4,5-d]azepin-7-carbonsäure-N-methylamid (amorph); aus 38.9 g 3-(Cyclopentylmethyl)-2,3,4,5-tetrahydro-1H-dibenz[2,3:6,7]oxepino [4,5-d]azepin-7-carbonsäure, 15,2 g Triäthylamin und 16,3 g Chlorameisensäureäthylester, dann 4,7 g Methylamin, mit Methylenchlorid als Lösungsmittel; das Methansulfonsäuresalz des Produkts schmilzt nach Kristallisieren aus Methanol bei 296-302°;

3-Methyl-2,3,4,5-tetrahydro-1H-dibenz[2,3:6,7]oxepino[4,5-d]azepin-7-carbonsäure-N,N-dimethylamid, Smp. 126-128° nach Kristallisieren aus einem Gemisch von Diäthyläther und n-Pentan; aus 32,1 g 3-Methyl-2,3,4,5-tetrahydro-1H-dibenz[2,3:6,7]oxepino[4,5-d]azepin -7-carbonsäure, 15,2 g Triäthylamin und 16,3 g Chlorameisensäureäthylester, dann 6,8 g Dimethylamin, mit Methylenchlorid als Lösungsmittel; das Methansulfonsäuresalz des Produkts schmilzt nach Kristallisieren aus Aceton bei 154-158°;

3-Methyl-2,3,4,5-tetrahydro-1H-dibenz[2,3:6,7]thiepino[4,5-d] azepin-7-carbonsäure-N,N-dimethylamid (amorph); aus 33,7 g 3-Methyl-2,3,4,5-tetrahydro-1H-dibenz[2,3:6,7]thiepino[4,5-d]azepin-7-carbonsäure, 15,2 g Triäthylamin und 16,3 g Chlorameisensäureäthylester, dann 6,8 g Dimethylamin mit Methylenchlorid als Lösungsmittel; das Fumarsäuresalz des Produkts schmilzt nach Kristallisieren aus einem Gemisch von absolutem Aethanol und Aceton bei 195-198°;

3-Methyl-7-pyrrolidinocarbonyl-2,3,4,5-tetrahydro-1H-dibenz [2,3:6,7]thiepino[4,5-d]azepin (amorph); aus 33,7 g 3-Methyl-2,3,4,5-tetrahydro-1H-dibenz[2,3:6,7]thiepino[4,5-d]azepin-7-carbonsäure, 15,2 g Triäthylamin und 16,3 g Chlorameisensäureäthylester, dann 10,7 g Pyrrolidin, mit Methylenchlorid als Lösungsmittel; das Fumarsäuresalz des Produkts schmilzt nach Kristallisieren aus einem Gemisch von absolutem Aethanol und Aceton bei 188-190°;

2-Methyl-2,3-dihydro-1H-dibenz[2,3:6,7]thiepino[4,5-c]pyrrol-5-carbonsäure-N,N-dimethylamid (amorph); aus 30,1 g 2-Methyl-2,3-dihydro-1H-dibenz[2,3:6,7]thiepino[4,5-c]pyrrol-5-carbonsäure, 15,2 g Triäthylamin und 16,3 g Chlorameisensäureäthylester, dann 6,8 g Dimethylamin, mit Methylenchlorid als Lösungsmittel; das Methansulfonsäuresalz des Produkts schmilzt nach Kristallisieren aus absolutem Aethanol bei 219-222°; und

2-Methyl-2,3-dihydro-1H-dibenz[2,3:6,7]thiepino[4,5-c]pyrrol-5-carbonsäure-N-methylamid, Smp. 170-175° nach Kristallisieren aus absolutem Aethanol; aus 30,1 g 2-Methyl-2,3-dihydro-1H-dibenz [2,3:6,7]thiepino[4,5-c]pyrrol-7-carbonsäure, 15,2 g Triäthylamin und 16,3 g Chlorameisensäureäthylester, dann 4,7 g Methylamin, mit Methylenchlorid als Lösungsmittel; das Methansulfonsäuresalz des Produkts schmilzt nach Kristallisieren aus Aethanol bei 270-273°.

Beispiel 6: 4,39 g 10,11-Bis-brommethyl-dibenz[b,f]thiepien-2-carboxamid werden in 400 ml absolutem Toluol bei 80° gelöst und innerhalb einer Stunde bei 43-45° zu einer Lösung von 155 g Methylamin in 400 ml Methanol getropft. Während dem Zutropfen wird zusätzlich Methylamingas eingeleitet. Man rührt das Reaktionsgemisch noch 1 Stunde bei 50° und destilliert anschliessend das Lösungsmittel und das überschüssige Methylamin ab. Den Rückstand versetzt man mit Wasser und extrahiert die erhaltene Suspension mit Methylenchlorid. Die organische Phase wird abgetrennt, mit Wasser gewaschen, über Kaliumcarbonat getrocknet und eingedampft. Den Rückstand löst man in 40 ml Aceton. Beim Abkühlen kristallisiert das 2-Methyl-2,3-dihydro-1H-dibenzo[2,3:6,7]thiepino-[4,5c]pyrrol-5-carboxamid und wird abgenutscht und getrocknet. Smp. 170-174°.

1,54 g der erhaltenen Base werden in 60 ml Aceton gelöst. Danach werden 0,48 g Methansulfosäure zugefügt. Das auskristallisierte Methansulfonat wird abgenutscht und getrocknet.
Smp. 220-223°.

Das Ausgangsmaterial kann auf folgende Weise hergestellt werden:

29,4 g 2-Cyano-10,11-dimethyl-dibenz[b,f]thiepin werden in 400 ml tert.-Butylalkohol vorgelegt. In diese Suspension trägt man unter Rühren 28 g pulverisiertes Kaliumhydroxid ein und kocht das Gemisch 1 Stunde unter Rückfluss. Danach destilliert man am Rotationsverdampfer 200 ml tert.-Butylalkohol ab. Zum Rückstand werden unter Rühren 100 ml Aether und 800 ml Wasser gegeben. Das ausgefallene 10,11-Dimethyl-dibenz[b,f]thiepin-2-carboxamid wird abgenutscht, mit Wasser gewaschen und aus 400 ml absolutem Alkohol umkristallisiert.
Smp. 195-200°.

2,81 g 10,11-Dimethyl-dibenz[b,f]thiepin-2-carboxamid werden in 900 ml Tetrachlorkohlenstoff bei 70° gelöst und danach 3,63 g N-Bromsuccinimid zugefügt. Unter Rühren und unter Belichten mit einer UV-Lampe wird das Gemisch 10 Minuten am Rückfluss gekocht. Man kühlt das Reaktionsgemisch auf 20° und nutscht das auskristallisierte rohe 10,11-Bis-brommethyl-dibenz[b,f]thiepin-2-carboxamid ab.
Das obige Produkt wird in Chloroform gelöst, zweimal mit 2n Natronlauge und danach dreimal mit Wasser gewaschen. Nach dem Trocknen über Natriumsulfat wird die Lösung auf 30 ml eingeengt, abgekühlt und genutscht.
Smp. 202-204°.

Beispiel 7: Tabletten, enthaltend 0.020 g des Methansulfonsäuresalzes von 2-Methyl-2,3-dihydro-1H-dibenz[2,3:6,7]thiepino[4,5-c]pyrrol-5-carboxamid, werden z.B. wie folgt hergestellt:

Zusammensetzung (für 10 000 Tabletten):

| | |
|---|---|
| 2-Methyl-2,3—dihydro-1H-dibenz [2,3:6,7]thiepino[4,5-c]azepin-5-carboxamid-methansulfonsäuresalz | 200,00 g |
| Lactose | 290,80 g |
| Kartoffelstärke | 274,70 g |
| Stearinsäure | 10,00 g |
| Talk | 200,00 g |
| Magnesiumstearat | 2,50 g |
| Kolloidales Siliciumdioxid | 32,00 g |
| Aethanol | q.s. |

Ein Gemisch des 2-Methyl-2,3-dihydro-1H—dibenz [2,3:6,7]thiepino-[4,5-c]pyrrol-5-carboxamid-methansulfonsäuresalzes, der Lactose und 194,70 g Kartoffelstärke wird mit einer äthanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen sein können.

Beispiel 8: Kapseln, enthaltend 0,025 g des 2-Methyl-2,3-dihydro-1H-dibenz[2,3:6,7]thiepino[4,5-c]pyrrol-5-carboxamid-methansulfonatsäuresalzes, können wie folgt hergestellt werden;

<u>Zusammensetzung</u> (für 1 000 Kapseln):

| | |
|---|---|
| 2-Methyl-2,3-dihydro-1H-dibenz [2,3:6,7]thiepino[4,5-c]pyrrol-5-carboxamid-methansulfonsäuresalz | 25,00 g |
| Lactose | 249,00 g |
| Gelatine | 2,00 g |
| Maisstärke | 10,00 g |
| Talk | 15,00 g |
| Wasser | q.s. |

Man mischt das 2-Methyl-2,3-dihydro-1H-dibenz [2,3:6,7]thiepino-[4,5-c]pyrrol-5-carboxamid-methansulfonsäuresalz mit der Lactose, befeuchtet die Mischung gleichmässig mit einer wässrigen Lösung der Gelatine und granuliert sie durch ein Sieb mit einer Maschenweite von 1,2-1,5 mm. Das Granulat mischt man mit der getrockneten Maisstärke und dem Talk und füllt Portionen von 300 mg in Hartgelatinekapseln (Grösse 1) ab.

Patentansprüche

1. Verbindungen der Formel

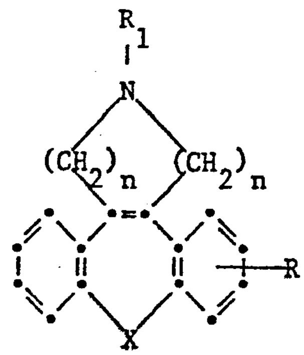

(I)

worin R eine gegebenenfalls veresterte oder amidierte Carboxygruppe, $R_1$ Wasserstoff oder einen gegebenenfalls substituierten aliphatischen oder cycloaliphatisch-aliphatischen Kohlenwasserstoffrest, X Epoxy oder Epithio, und n 1 oder 2 bedeuten, und Salze davon.

2. Verbindungen gemäss Anspruch 1 der Formel I, worin R für Carboxy, Niederalkoxycarbonyl, Carbamoyl, Niederalkylcarbamoyl, Diniederalkylcarbamoyl, Niederalkylenaminocarbonyl mit 5 bis 8 Ringatomen, Oxaniederalkylenaminocarbonyl mit 6 Ringatomen, Thianiederalkylenaminocarbonyl mit 6 Ringatomen oder Azaniederalkylenaminocarbonyl mit 6 bis 8 Ringatomen steht, wobei der Azastickstoff gegebenenfalls durch Niederalkyl, Hydroxyniederalkyl, Niederalkoxyniederalkyl oder Niederalkanoyloxyniederalkyl substituiert sein kann, wobei in solchen substituierten Niederalkylresten ein Substituent durch mindestens 2 Kohlenstoffatome vom Azastickstoffatom getrennt ist, $R_1$ Wasserstoff, Niederalkyl, Hydroxyniederalkyl, Niederalkoxyniederalkyl, Niederalkanoyloxyniederalkyl, Halogenniederalkyl, Niederalkenyl, Niederalkinyl oder Cycloalkylniederalkyl bedeutet, wobei in substituierten Niederalkylresten ein Substituent vorzugsweise durch mindestens 2 Kohlenstoffatome vom Ringstickstoffatom getrennt ist, in ungesättigten Resten die Doppel- bzw. Dreifachbindung in höherer als der 1-Stellung vorliegt, und Cycloalkyl 3 bis 8 Ringatome enthält, wobei mit "nieder" bezeichnete Reste

bis und mit 7 Kohlenstoffatome enthalten, X Epoxy oder Epithio bedeutet, und n für 1 oder 2 steht, und Salze davon.

3. Verbindungen gemäss Anspruch 1 der Formel I, worin R Carboxy, Niederalkoxycarbonyl, Carbamoyl, Niederalkylcarbamoyl, Diniederalkylcarbonyl, oder Niederalkylenaminocarbonyl, worin Niederalkylenamino 5 oder 6 Ringatome enthält, bedeutet, $R_1$ Wasserstoff, Niederalkyl, Hydroxyniederalkyl oder Niederalkoxyniederalkyl, worin Hydroxy bzw. Niederalkoxy durch mindestens 2 Kohlenstoffatome vom Ringstickstoffatom getrennt ist, Niederalkenyl oder Niederalkinyl, worin sich die Doppel- bzw. Dreifachbindung in einer höheren als der 1-Stellung befindet, oder Cycloalkylniederalkyl darstellt, worin Cycloalkyl 3 bis 8 Ringkohlenstoffatome enthält, wobei mit "nieder" bezeichnete Reste bis und mit 4 Kohlenstoffatome enthalten, X für Epoxy oder Epithio steht, und n 1 oder 2 bedeutet, und Salze davon.

4. Verbindungen gemäss Anspruch 1 der Formel

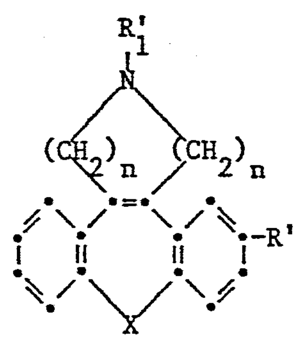

(Ia) ,

worin R' Carboxy, Niederalkoxycarbonyl, Carbamoyl, Niederalkylcarbamoyl, Diniederalkylcarbamoyl oder Niederalkylenaminocarbonyl, worin Niederalkylenamino 5 oder 6 Ringatome enthält, bedeutet, $R_1'$ für Wasserstoff, Niederalkyl oder Cycloalkylniederalkyl steht, worin Cycloalkyl 3 bis 6 Ringkohlenstoffatome hat, wobei mit "nieder" bezeichnete Reste bis und mit 4 Kohlenstoffatome enthalten, X Epoxy oder Epithio bedeutet, und n für 1 oder 2 steht, und Salze davon.

5. Verbindungen gemäss Anspruch 1 der Formel Ia des Anspruches 4, worin R' für Carbamoyl, Niederalkylcarbamoyl, worin Niederalkyl bis und mit 4 Kohlenstoffatome aufweist, Diniederalkylcarbamoyl, worin Niederalkyl bis und mit 4 Kohlenstoffatome aufweist, oder Alkylenaminocarbonyl, worin Alkylenamino 5 oder 6 Ringatome enthält, steht, $R_1'$ Niederalkyl mit bis und mit 4 Kohlenstoffatomen, oder Cycloalkylniederalkyl, worin Cycloalkyl 3 bis 6 Ringkohlenstoffatome aufweist, und Niederalkyl bis und mit 4 Kohlenstoffatome hat, X Epoxy oder Epithio darstellt, und n 1 oder 2 bedeutet, und Salze davon.

6. 2-Methyl-2,3-dihydro-1H-dibenz[2,3:6,7]thiepino[4,5-c]pyrrol-5-carboxyamid und Salze davon.

7. Verbindungen gemäss Anspruch 1 mit neuroleptischen Eigenschaften.

8. Verbindungen der Formel I gemäss Anspruch 1 oder pharmazeutisch verwendbare Salze davon zur Anwendung bei der Behandlung des menschlichen oder tierischen Körpers.

9. Pharmazeutische Präparate enthaltend eine der Verbindungen der Formel I gemäss Anspruch 1 oder pharmazeutisch verwendbare Salze davon.

10. Verwendung der Verbindungen der Formel I gemäss Anspruch 1 oder pharmazeutisch verwendbarer Salze davon zur Herstellung von pharmazeutischen Präparaten.

11. Verwendung der Verbindungen der Formel I gemäss Anspruch 1 oder pharmazeutisch verwendbarer Salze davon zur Behandlung des menschlichen oder tierischen Körpers.

12. Verfahren zur Herstellung von Verbindungen der Formel I gemäss Anspruch 1 oder Salzen davon, dadurch gekennzeichnet, dass man

(a) in einer Verbindung der Formel

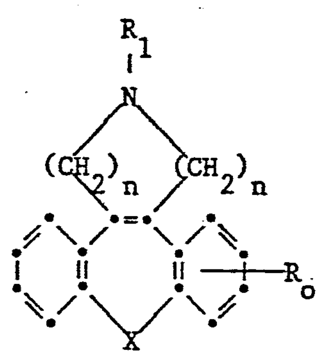

(II),

worin $R_o$ einen in die gegebenenfalls veresterte oder amidierte Carboxygruppe R überführbaren Rest darstellt, oder in einem Salz davon $R_o$ in die Gruppe R überführt, oder

(b) eine Verbindung der Formel

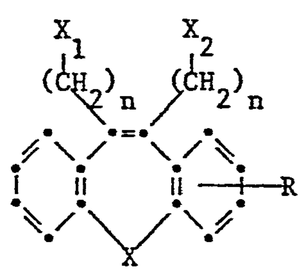

(III) ,

worin eine der Gruppen $X_1$ und $X_2$ den Rest der Formel $-NH-R_1$ (IIIa) und die andere eine reaktionsfähige veresterte Hydroxygruppe bedeutet, ringschliesst, und wenn erwünscht, eine erhältliche Verbindung der Formel I in eine andere Verbindung der Formel I überführt, und/oder, wenn erwünscht eine erhaltenes Salz in die freie Verbindung oder in ein anderes Salz überführt, und/oder, wenn erwünscht, eine erhaltene freie Verbindung in ein Salz überführt, und/oder, wenn erwünscht, ein erhaltenes Isomerengemisch in die einzelnen Isomeren auftrennt.

13. Die nach dem Verfahren des Anspruchs 12 erhältlichen Verbindungen.

FO 7.4/HRS/mg*

Europäisches Patentamt

# EUROPÄISCHER RECHERCHENBERICHT

0125484
Nummer der Anmeldung

EP 84 10 3951

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A,D | EP-A-0 007 450 (CIBA-GEIGY) * Ansprüche 1, 14, 15 * | 1,9,10 | C 07 D 491/044<br>C 07 D 495/04<br>A 61 K 31/40<br>A 61 K 31/55 |
| A | DE-A-2 506 155 (CIBA-GEIGY) * Ansprüche 1, 39, 41 * | 1,9,10,12 | |
| | | | **RECHERCHIERTE SACHGEBIETE (Int. Cl. 3)** |
| | | | A 61 K 31/40<br>A 61 K 31/55<br>C 07 D 491/04<br>C 07 D 491/044<br>C 07 D 495/04 |

Der vorliegende Recherchenbericht wurde für alle Patentansprüche erstellt.

| Recherchenort | Abschlußdatum der Recherche | Prüfer |
|---|---|---|
| BERLIN | 19-07-1984 | KNAACK M |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument